# EUROPEAN PATENT APPLICATION

(11) **EP 4 268 833 A1**
(43) Date of publication of application: **01.11.2023**
(21) Application number: 21911060.8
(22) Date of filing: 24.12.2021
(51) Int. Cl.: A61K 35/02, A23L 2/00, A23L 2/52, A23L 33/10, A61K 8/24, A61K 8/25, A61K 33/12, A61K 33/42, A61P 17/00, A61P 43/00, A61Q 19/00

(54) **CERAMIDE SYNTHESIS PROMOTER**

(30) Priority: 25.12.2020 JP 2020217526
(71) Applicant: A.I.System Products Corp., Kasugai-shi, Aichi 480-0305 (JP)
(72) Inventor: OKAJIMA Masahiro, Kasugai-shi, Aichi 480-0305 (JP); IKEDA Mitsuo, Kasugai-shi, Aichi 480-0305 (JP); YAMAMOTO Hiroyuki, Kitaadachi-gun, Saitama 362-0806 (JP)
(74) Representative: Hasegawa, Kan
(86) International application number: PCT/JP2021/048218
(87) International publication number: WO 2022/138923

(57) **Abstract**

It is an object of the present invention to provide a novel agent for promoting ceramide synthesis. The present invention relates to an agent for promoting ceramide synthesis comprising ionized alkaline water obtained by electrolyzing an aqueous solution comprising a mineral salt containing silicon and phosphorus.

## Description

### Technical Field

The present invention relates to an agent for promoting ceramide synthesis.

### Background Art

Skin ceramides are biosynthesized by the formation of sphingosine backbones from kinds of amino acids, L-serine and palmitic acid, as starting materials in epidermal keratinocytes, followed by various enzymatic reactions. Healthy metabolism of ceramides is impaired by aging, ultraviolet exposure, dry skin, rough skin texture, atopic dermatitis, senile xerosis, psoriasis, and the like. This results in a decrease in the amount of ceramides in each layer, which may lead, for example, to a reduction in the skin moisturizing function and barrier function. It has recently been reported that acylceramide, which has a structure in which linoleic acid is bound to the ω-hydroxyl group of ceramides and has a fatty acid chain length of C28 or more, is extremely hydrophobic and essential for the skin barrier function (Non Patent Literature 1).

Thus, promoting the production of ceramides, particularly acylceramide, is expected to improve or normalize the skin barrier function.

Substances for promoting the production of ceramides, particularly acylceramide, that can be used in skin external preparations, foods and beverages, and the like have recently been under active research and development. However, sufficient results have not been obtained, and there is a desire for the development of a substance having a safe and effective ceramide synthesis-promoting effect.

### Citation List

### Non Patent Literature

Non Patent Literature 1: YAKUGAKU ZASSHI 137(10) 1201-1208 (2017)

### Summary of Invention

### Technical Problem

It is an object of the present invention to provide a novel agent for promoting ceramide synthesis.

### Solution to Problem

As a result of extensive research by the inventors of the present invention to develop a novel agent for promoting ceramide synthesis, the inventors have found that ionized alkaline water produced using a specific method has the effect of promoting the expression of genes involved in ceramide synthesis, and ceramide synthesis is promoted by using this ionized alkaline water. The present invention has been completed based on this finding.

In summary, the present invention is as set forth below.

### Item 1.

An agent for promoting ceramide synthesis comprising ionized alkaline water obtained by electrolyzing an aqueous solution comprising a mineral salt containing silicon and phosphorus.

### Item 2.

The agent for promoting ceramide synthesis according to item 1, wherein the ionized alkaline water contains 0.4 to 400 mass ppm of the silicon and 2 to 800 mass ppm of the phosphorus.

### Item 3.

The agent for promoting ceramide synthesis according to item 1 or 2, wherein the ionized alkaline water further contains at least one mineral selected from the group consisting of calcium, potassium, sodium, and magnesium.

### Item 4.

The agent for promoting ceramide synthesis according to any one of items 1 to 3, wherein the ionized alkaline water further contains chlorine.

### Item 5.

The agent for promoting ceramide synthesis according to any one of items 1 to 4, wherein the ionized alkaline water has a pH of 10 to 12.5.

### Item 6.

The agent for promoting ceramide synthesis according to any one of items 1 to 5, wherein the ionized alkaline water has a surface tension of 55 to 68 mN/m.

### Item 7.

The agent for promoting ceramide synthesis according to any one of items 1 to 6, wherein the ionized alkaline water is obtained by:
an electrolyzing step of electrolyzing an aqueous solution comprising water and a mineral salt containing silicon and phosphorus; and
an electron supplying step of mixing cathode side-alkaline water obtained in the preceding step with a mineral salt containing silicon and phosphorus to obtain a mixture, and supplying electrons to the mixture.

### Item 8.

The agent for promoting ceramide synthesis according to any one of items 1 to 7, wherein the agent for promoting ceramide synthesis comprises an agent for promoting ceramide synthesis gene expression.

### Item 9.

The agent for promoting ceramide synthesis according to any one of items 1 to 8, wherein the agent for promoting ceramide synthesis gene expression comprises at least one agent for promoting gene expression selected from the group consisting of an agent for promoting ELOVL4 gene expression, an agent for promoting ELOVL7 gene expression, an agent for promoting CerS3 gene expression, and an agent for promoting SPTLC2 gene expression.

### Item 10.

The agent for promoting ceramide synthesis according to any one of items 1 to 9, wherein the agent for promoting ceramide synthesis comprises the agent for promoting ELOVL4 gene expression.

### Item 11.

The agent for promoting ceramide synthesis according to any one of items 1 to 9, wherein the agent for promoting ceramide synthesis comprises the agent for promoting ELOVL7 gene expression.

### Item 12.

The agent for promoting ceramide synthesis according to any one of items 1 to 9, wherein the agent for promoting ceramide synthesis comprises the agent for promoting CerS3 gene expression.

### Item 13.

The agent for promoting ceramide synthesis according to any one of items 1 to 9, wherein the agent for promoting ceramide synthesis comprises the agent for promoting SPTLC2 gene expression.

### Item 14.

A pharmaceutical preparation comprising the agent for promoting ceramide synthesis according to any one of items 1 to 13.

### Item 15.

A skin external preparation comprising the agent for promoting ceramide synthesis according to any one of items 1 to 13.

### Item 16.

A cosmetic preparation comprising the agent for promoting ceramide synthesis according to any one of items 1 to 13.

### Item 17.

A food or beverage composition comprising the agent for promoting ceramide synthesis according to any one of items 1 to 13.

### Item 18.

A food or beverage composition for promoting ceramide synthesis according to any one of items 1 to 13.

### Item 19.

Use of ionized alkaline water obtained by electrolyzing an aqueous solution comprising a mineral salt containing silicon and phosphorus, for promoting ceramide synthesis.

### Item 20.

The use according to item 19, wherein the ionized alkaline water contains 0.4 to 400 mass ppm of the silicon and 2 to 800 mass ppm of the phosphorus.

### Item 21.

The use according to item 19 or 20, wherein the ionized alkaline water further contains at least one mineral selected from the group consisting of calcium, potassium, sodium, and magnesium.

### Item 22.

The use according to any one of items 19 to 21, wherein the ionized alkaline water further contains chlorine

### Item 23.

The use according to any one of items 19 to 22, wherein the ionized alkaline water has a pH of 10 to 12.5.

### Item 24.

The use according to any one of items 19 to 23, wherein the ionized alkaline water has a surface tension of 55 to 68 mN/m.

### Item 25.

The use according to any one of items 19 to 24, wherein the ionized alkaline water is obtained by:
an electrolyzing step of electrolyzing an aqueous solution comprising water and a mineral salt containing silicon and phosphorus; and
an electron supplying step of mixing cathode side-alkaline water obtained in the preceding step with a mineral salt containing silicon and phosphorus to obtain a mixture, and supplying electrons to the mixture.

### Item 26.

A method for promoting ceramide synthesis,
the method comprising using ionized alkaline water obtained by electrolyzing an aqueous solution comprising a mineral salt containing silicon and phosphorus.

### Item 27.

The method according to item 26, wherein the ionized alkaline water contains 0.4 to 400 mass ppm of the silicon and 2 to 800 mass ppm of the phosphorus.

### Item 28.

The method according to item 26 or 27, wherein the ionized alkaline water further contains at least one mineral selected from the group consisting of calcium, potassium, sodium, and magnesium.

### Item 29.

The method according to any one of items 26 to 28, wherein the ionized alkaline water further contains chlorine.

### Item 30.

The method according to any one of items 26 to 29, wherein the ionized alkaline water has a pH of 10 to 12.5.

### Item 31.

The method according to any one of items 26 to 30, wherein the ionized alkaline water has a surface tension of 55 to 68 mN/m.

### Item 32.

The method according to any one of items 26 to 31, wherein the ionized alkaline water is obtained by:
an electrolyzing step of electrolyzing an aqueous solution comprising water and a mineral salt containing silicon and phosphorus; and
an electron supplying step of mixing cathode side-alkaline water obtained in the preceding step with a mineral salt containing silicon and phosphorus to obtain a mixture, and supplying electrons to the mixture.

### Item 33.

A method for producing an agent for promoting ceramide synthesis, comprising the step of:
electrolyzing an aqueous solution comprising a mineral salt containing silicon and phosphorus.

### Item 34.

The method for producing an agent for promoting ceramide synthesis according to item 33, wherein the ionized alkaline water contains 0.4 to 400 mass ppm of the silicon and 2 to 800 mass ppm of the phosphorus.

### Item 35.

The method for producing an agent for promoting ceramide synthesis according to item 33 or 34, wherein the ionized alkaline water further contains at least one mineral selected from the group consisting of calcium, potassium, sodium, and magnesium.

### Item 36.

The method for producing an agent for promoting ceramide synthesis according to any one of items 33 to 35, wherein the ionized alkaline water further contains chlorine.

### Item 37.

The method for producing an agent for promoting ceramide synthesis according to any one of items 33 to 36, wherein the ionized alkaline water has a pH of 10 to 12.5.

### Item 38.

The method for producing an agent for promoting ceramide synthesis according to any one of items 33 to 37, wherein the ionized alkaline water has a surface tension of 55 to 68 mN/m.

### Item 39.

The method for producing an agent for promoting ceramide synthesis according to any one of items 33 to 38, wherein the ionized alkaline water is obtained by:
an electrolyzing step of electrolyzing an aqueous solution comprising water and a mineral salt containing silicon and phosphorus; and
an electron supplying step of mixing cathode side-alkaline water obtained in the preceding step with a mineral salt containing silicon and phosphorus to obtain a mixture, and supplying electrons to the mixture.

At present, it is impossible or impractically difficult to completely specify the agent for promoting ceramide synthesis, the agent for promoting gene expression, the pharmaceutical preparation, the skin external preparation, the cosmetic preparation, and the food or beverage composition, based on their product structure; therefore, each of these product inventions is specified in a product-by-process claim.

### Advantageous Effects of Invention

According to the present invention, there is provided an agent for promoting ceramide synthesis comprising ionized alkaline water obtained by electrolyzing an aqueous solution comprising a mineral salt containing silicon and phosphorus. The ionized alkaline water obtained by electrolyzing an aqueous solution comprising a mineral salt containing silicon and phosphorus has the effect of promoting the expression of the ELOVL4, ELOVL7, CerS3, and SPTLC2 genes, involved in the synthesis of ceramides, particularly acylceramide, and thus, is capable of promoting the synthesis of ceramides. The agent for promoting ceramide synthesis of the present invention is thus effective for preventing or treating skin disorders induced by a reduction in ceramides.

### Brief Description of Drawings

Fig. 1 is a schematic diagram for illustrating an apparatus for producing ionized alkaline water.
Fig. 2 shows the gene sequence of human ELOVL4 (SEQ ID NO: 1).
Fig. 3 shows the gene sequence of human ELOVL7 (SEQ ID NO: 2).
Fig. 4 shows the gene sequence of human CerS3 (SEQ ID NO: 3).
Fig. 5A shows the gene sequence of human SPTLC2 (SEQ ID NO: 4).
Fig. 5B shows the gene sequence of human SPTLC2 (SEQ ID NO: 4), continued from Fig. 5A.
Fig. 6 is a graph showing the ELOVL4 mRNA expression level obtained with the ionized alkaline water.
Fig. 7 is a graph showing the ELOVL7 mRNA expression level obtained with the ionized alkaline water.
Fig. 8 is a graph showing the CerS3 mRNA expression level obtained with the ionized alkaline water.
Fig. 9 is a graph showing the SPTLC2 mRNA expression level obtained with the ionized alkaline water.
Fig. 10 shows the results of evaluating variations in the expression of the ELOVL4 and CerS3 proteins by Western blotting.
Fig. 11 shows the results of evaluation by thin-layer ch0015 romatography.
Fig. 12 is a graph showing the increase ratio of the color intensity of the GlcCer region at each concentration in Fig. 11.
Fig. 13 is a graph showing the increase ratio of the color intensity of the Cer region at each concentration in Fig. 11.

### Description of Embodiments

The agent for promoting ceramide synthesis of the present invention comprises ionized alkaline water obtained by electrolyzing an aqueous solution comprising a mineral salt containing silicon and phosphorus (hereinafter also simply referred to as the "ionized alkaline water"). The ionized alkaline water obtained by electrolyzing an aqueous solution comprising a mineral salt containing silicon and phosphorus has the effect of promoting the expression of genes involved in ceramide synthesis, and is capable of promoting the synthesis of ceramides; thus, the agent for promoting ceramide synthesis of the present invention is effective for preventing or treating skin disorders induced by a reduction in ceramides.

The ionized alkaline water obtained by electrolyzing an aqueous solution comprising a mineral salt containing silicon and phosphorus contains silicon and phosphorus. Specifically, the ionized alkaline water contains silicon (Si) in an amount of about 0.4 to 400 mass ppm (mg/L), preferably about 0.8 to 390 mass ppm, more preferably about 1.0 to 380 mass ppm, and contains phosphorus (P) in an amount of about 2 to 800 mass ppm, preferably about 4 to 780 mass ppm, more preferably about 10 to 770 mass ppm. Elemental analysis is performed by inductively coupled plasma (ICP) optical emission spectrometry (SPECTRO ACROS II from Hitachi High-Tech Science Corporation).

The ionized alkaline water obtained by electrolyzing an aqueous solution comprising a mineral salt containing silicon and phosphorus may contain other elements besides phosphorus and silicon, and may, for example, contain calcium (Ca) in an amount of about 0.0001 to 900 mass ppb, preferably about 0.001 to 800 mass ppm, more preferably about 0.01 to 700 mass ppm, may contain potassium (K) in an amount of about 0.0005 to 3000 mass ppm, preferably about 1 to 2900 mass ppm, more preferably about 5 to 2800 mass ppm, may contain magnesium (Mg) in an amount of about 0.0001 to 300 mass ppb, preferably about 0.001 to 250 mass ppm, more preferably about 0.01 to 200 mass ppm, and may contain sodium (Na) in an amount of about 34 to 8000 mass ppm, preferably about 40 to 7900 mass ppm, more preferably about 50 to 7800 mass ppm. The ionized alkaline water obtained by electrolyzing an aqueous solution comprising a mineral salt containing silicon and phosphorus may further contain chlorine (Cl), and may, for example, contain chlorine (Cl) in an amount of about 1.5 to 450 mg/kg, preferably about 1.5 to 440 mg/kg, more preferably about 1.5 to 430 mg/kg.

The silicon contained in the ionized alkaline water may be present in the form of silicate ions, for example, H₃SiO₄⁻, H₅Si₂O₇⁻, H₅S₁₃O₉⁻, and the like.

The phosphorus contained in the ionized alkaline water may be present in the form of phosphate ions, for example, H₂PO₄⁻, H₃P₂O₇⁻, H₄P₃O₁₀⁻, and the like.

The ionized alkaline water obtained by electrolyzing an aqueous solution comprising a mineral salt containing silicon and phosphorus typically has a pH of about 10 to 12.5. The ionized alkaline water typically has a surface tension of 70 mN/m or less, preferably 50 to 69 mN/m, more preferably about 55 to 68 mN/m. Furthermore, the ionized alkaline water physically contains an excess of electrons.

The ionized alkaline water to be used in the present invention can be obtained by an electrolyzing step of electrolyzing an aqueous solution comprising water and a mineral salt containing silicon and phosphorus; and an electron supplying step of mixing cathode side-alkaline water obtained in the preceding step with a mineral salt containing silicon and phosphorus to obtain a mixture, and supplying electrons to the mixture. The mineral salt containing silicon and phosphorus may, for example, be an electrolyte containing silicon and phosphorus obtained from seawater. Any seawater may be used without limitation; for example, seawater in Japan may be used. The mineral salt containing silicon and phosphorus may be added in an amount of about 0.001 to 3% by mass based on the amount of the water used.

The method for producing the ionized alkaline water described above preferably includes, in addition to the above-described steps, a deoxidizing step of subjecting the aqueous solution comprising water and a mineral salt containing silicon and phosphorus to a deoxidation treatment to reduce the dissolved oxygen concentration to 1 ppm or less; and a stabilizing step of applying a pressure of 1 to 12 kg/cm² to the mixture of the cathode-side alkaline water with the mineral salt containing silicon and phosphorus. The mineral salt containing silicon and phosphorus to be mixed with the cathode-side alkaline water may be the same as the mineral salt containing silicon and phosphorus used in the electrolyzing step, and may, for example, be a mineral salt containing silicon and phosphorus collected from seawater in Japan.

The method for producing the ionized alkaline water will be described, hereinafter.

The first step is the electrolyzing step of electrolyzing an aqueous solution comprising water and a mineral salt containing silicon and phosphorus.

The water to be used as raw material water may be pure water, deionized water, or the like. A mineral salt containing silicon and phosphorus obtained from seawater, for example, as a supporting electrolyte, is dissolved in the water. The water is preferably water that has been subjected to a deoxidation treatment to reduce the concentration of dissolved oxygen in the water to 1 ppm or less. Deoxidation treatments are classified as physical treatment methods and chemical treatment methods. Conventionally, a chemical treatment method is employed alone, or a physical treatment method and a chemical treatment method are employed in combination. Examples of physical treatment methods include a degassing treatment with a heating degassing device, a membrane degassing device, or the like. Examples of chemical treatment methods include a method in which a deoxidizer, such as hydrazine, sodium sulfite, or a saccharide (such as glucose), is added. In the deoxidation treatment, for example, these deoxidizers may be added to the water.

The electrolysis is preferably performed in an enclosed space, such as an electrolytic cell. The atmosphere in the electrolytic cell is preferably an inert atmosphere, such as nitrogen or carbon dioxide. Furthermore, instead of using the water that has been previously subjected to a deoxidation treatment to reduce the concentration of dissolved oxygen to 1 ppm or less, or in addition to using this previously deoxidized water, a deoxidizer may be introduced into the electrolytic cell at the time of the electrolysis in the electrolytic cell. The electrical energy applied in the electrolysis is preferably 1000 to 3000 W.

The second step is the electron supplying step of mixing cathode side-alkaline water obtained in the first step with a mineral salt containing silicon and phosphorus to obtain a mixture, and supplying electrons to the mixture.

Preferably, the cathode-side alkaline water obtained by electrolysis is drawn from the electrolytic cell and supplied into a mixing tank. The mixing tank preferably includes a means for supplying the mineral salt containing silicon and phosphorus obtained from seawater and an electron supply means for supplying electricity to the alkaline water.

Examples of raw materials containing silicon (or mineral salts containing silicon) include alkali metal silicates, such as sodium silicate and potassium silicate; alkaline earth metal silicates, such as calcium silicate; and magnesium silicate. The silicon content in the mixture is preferably about 0.005 to 5% by mass, and more preferably 0.01 to 1% by mass, based on the total amount of the mixture taken as 100% by mass.

Examples of raw materials containing phosphorus (or mineral salts containing phosphorus) include alkali metal phosphates, such as sodium phosphate and potassium phosphate; alkaline earth metal phosphates, such as calcium phosphate; and magnesium phosphate. The phosphorus content in the mixture is preferably about 0.005 to 5% by mass, and more preferably 0.01 to 1% by mass, based on the total amount of the mixture taken as 100% by mass.

The mass ratio between the mineral salt containing silicon and the mineral salt containing phosphorus to be added to the alkaline water is preferably 1:0.5 to 1.5.

Examples of the electron supply means include a cathode terminal. By contacting the cathode-side alkaline water obtained by electrolysis with a cathode terminal, electrons can be supplied to the alkaline water. The cathode electron discharges electrons to supply many electrons to the alkaline water.

A direct current is preferably applied to the cathode terminal. The voltage of the direct current to be applied to the cathode terminal is, for example, 10 to 1000 V, preferably 50 to 300 V, and more preferably 100 to 300 V. It is believed that the supply of electrons to the mixture allows the resulting ionized alkaline water to effectively exhibit the effect of expressing a ceramide synthesis gene.

Preferably, a direct current with a voltage of 100 to 300 V is applied to the mixing tank, and the amount of electricity discharged from the cathode electron is 1000 to 3000 W.

A pressure of preferably 1 to 12 kg/cm² (98 to 1177 kPa), more preferably 2 to 6 kg/cm² (196 to 588 kPa), is applied to the mixing tank. The application of the pressure improves the stability of the ionized alkaline water.

The atmosphere in the mixing tank is preferably an inert atmosphere, such as nitrogen or carbon dioxide. Furthermore, a deoxidizer may be introduced into the mixing tank. The mixing tank preferably includes a thermal insulating means for thermally insulating the mixing tank from the ambient temperature so as not to be affected by the ambient temperature, and a temperature adjusting means for adjusting the internal temperature of the mixing tank in the range of -5 to 25°C. The temperature of the mixture is preferably 0 to 10°C.

The mixing tank is preferably placed in an isolated tank isolated from the external atmosphere, and the isolated tank is preferably filled with nitrogen or carbon dioxide. This can prevent deterioration of the ionized alkaline water, leading to the maintenance of performance over a long period of time.

The ionized alkaline water as a final product is obtained after stabilizing the ionized alkaline water in the mixing tank for 24 to 36 hours. The resulting ionized alkaline water can be directly drawn through an ionized alkaline water outlet. Alternatively, the ionized alkaline water may be mixed with an alcohol, and the mixture may be drawn through an ionized alkaline water mixture outlet. The alcohol is preferably an alcohol that is highly miscible with water, for example, methanol, ethanol, and isopropyl alcohol.

An apparatus that can be used to produce the ionized alkaline water will be described, hereinafter.

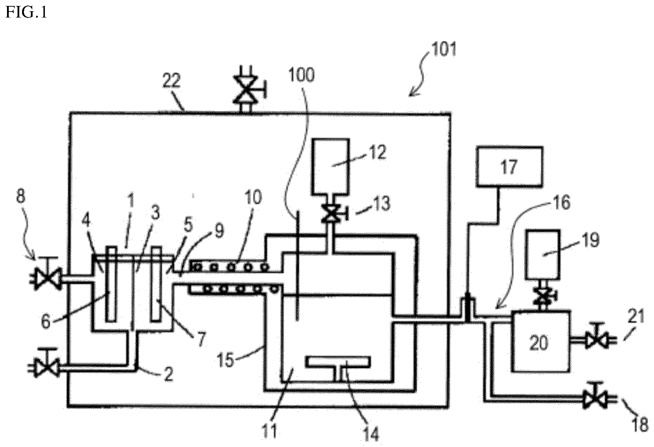

Fig. 1 is a schematic diagram for illustrating an apparatus for producing ionized alkaline water. An ionized alkaline water producing apparatus 101 includes an electrolytic cell 1 and a mixing tank 11. The electrolytic cell 1 and the mixing tank 11 are connected through an alkaline water delivery pipe 9. The electrolytic cell 1 is connected with a water conduit 2. Through the water conduit 2, raw material water (aqueous solution comprising water and a mineral salt containing silicon and phosphorus) is supplied to the electrolytic cell 1. The electrolytic cell 1 has an anode chamber 4 and a cathode chamber 5 separated by a diaphragm 3. The anode chamber 4 has an anode 6, and the cathode chamber 5 has a cathode 7. After an electrolytic current is applied to both electrodes, acidic water is drawn through an acidic water discharge pipe 8 connected to the anode chamber 4, and alkaline water is drawn through an alkaline water delivery pipe 9 connected to the cathode chamber 5. While passing through the alkaline water delivery pipe 9, the alkaline water is cooled by a cooling device 10 having a cooling tube in which a refrigerant is circulated, and then introduced into the mixing tank 11.

The mixing tank 11 is connected with the alkaline water delivery pipe 9, a raw material liquid reservoir 12, and an outlet pipe 16. A raw material liquid is supplied to the mixing tank 11 from the raw material liquid reservoir 12 through an inflow adjusting device 13. The supply amount of the raw material liquid is adjusted according to the amount of electricity supplied, for example. The raw material liquid may be a mineral salt containing silicon and phosphorus collected from seawater in Japan. The mixing tank 11 has a stirring device 14, which homogeneously mixes the ionized alkaline water with the raw material liquid.

A cathode terminal 100 is placed in the mixing tank 11. A direct current at 200 V is applied to the cathode terminal 100. The amount of electricity discharged from the cathode terminal 100 is preferably 1000 to 3000 W.

The mixing tank 11 is thermally insulated by a thermal insulating means 15 so as not to be affected by the ambient temperature, and is adjusted to an internal temperature of about 15 to 25°C by a temperature adjusting means (not shown). The temperature of the mixing tank 11 is preferably about 0 to 10°C.

The pH of the ionized alkaline water delivered from the mixing tank 11 is measured by a pH measuring means 17 provided at the outlet pipe 16. The ionized alkaline water drawn from the mixing tank 11 preferably has a pH of about 10 to 12.5.

The outlet pipe 16 connected to the mixing tank 11 branches into an ionized alkaline water outlet pipe 18 and an ionized alkaline water mixture outlet pipe 21. The ionized alkaline water in the mixing tank 11 is directly drawn through the ionized alkaline water outlet pipe 18. The ionized alkaline water may be mixed with an alcohol to form an ionized alkaline water mixture. In this case, the ionized alkaline water and the alcohol may be mixed in an alcohol mixing tank 20, into which an alcohol reservoir 19 introduces the alcohol, and the resulting mixture may be drawn as an ionized alkaline water mixture through the ionized alkaline water mixture outlet pipe 21. The alcohol to be mixed with the ionized alkaline water is preferably an alcohol that is highly miscible with water, for example, methanol, ethanol, and isopropyl alcohol.

The ionized alkaline water producing apparatus 101 is housed in an isolated chamber 22 so as to be isolated from the external air. The isolated chamber 22 is connected to the external atmosphere through a venting device that is filled with carbon dioxide, an oxygen scavenger, or the like, or purged with nitrogen. Placing the ionized alkaline water producing apparatus 101 in the isolated chamber 22 can prevent deterioration of the ionized alkaline water, leading to the maintenance of performance over a long period of time.

The ionized alkaline water obtained by electrolyzing an aqueous solution comprising a mineral salt containing silicon and phosphorus may be a commercially available product. Examples of commercially available products include trade names S-100 and S-100G (both from A. I. SYSTEM PRODUCTS CORP.).

The ionized alkaline water produced using the above-described method has an excellent effect of promoting ceramide synthesis gene expression and thus, can be used as an agent for promoting ceramide synthesis gene expression.

The ionized alkaline water obtained by electrolyzing an aqueous solution comprising a mineral salt containing silicon and phosphorus exhibits the effect of promoting the expression of genes involved in the synthesis of ceramides, particularly the synthesis of acylceramide. Specifically, the ionized alkaline water exhibits the effect of promoting the expression of the ELOVL (very long chain fatty acid elongase) 4 gene, which is essential for the synthesis of C28 or higher fatty acids abundantly found in the epidermis (effect of promoting ELOVL4 gene expression); the effect of promoting the expression of the ELOVL7 gene, which plays a critical role in the step of synthesizing C16, C18, and C20 fatty acids (effect of promoting ELOVL7 gene expression); and the effect of promoting the expression of the CerS3 gene, which is active with ultra-long chain acyl-CoA, among ceramide synthetases, which are enzymes that synthesize ceramides by binding acyl-CoA to a long-chain base (effect of promoting CerS3 gene expression). The ionized alkaline water also exhibits the effect of promoting the expression of the SPTLC2 gene, which is a subunit of serine palmitoyl transferase (SPT), which is an enzyme involved in ceramide synthesis in epidermal keratinocytes (effect of promoting SPTLC2 gene expression). Thus, the ionized alkaline water containing silicon and phosphorus can be used as an agent for promoting ELOVL4 gene expression, an agent for promoting ELOVL7 gene expression, an agent for promoting CerS3 gene expression, and an agent for promoting SPTLC2 gene expression.

While the agent for promoting ceramide synthesis of the present invention can be used alone, it may also contain additives. Examples of additives include sodium chloride, various vitamins, and glucose. The agent for promoting ceramide synthesis of the present invention may be added to various compositions, such as a pharmaceutical preparation, a quasi-drug, a food or beverage, and a cosmetic preparation, to give compositions having the effect of promoting the synthesis of ceramides.

Forms of the food or beverage containing the agent for promoting ceramide synthesis of the present invention are not limited. Specific forms of the food or beverage include, for example, general foods, general beverages, supplements, health foods, foods with function claims, specified health foods or other foods with health claims and foods for specified uses, soft drinks, tea beverages, drinks, alcoholic beverages such as wine, confectioneries, rice, bread, noodles, cooked dishes, and seasonings.

Forms of the pharmaceutical preparation or the quasi-drug containing the agent for promoting ceramide synthesis of the present invention are not limited. Specific forms include, for example, internal preparations and external preparations, with a skin external preparation being preferred.

Forms of the cosmetic preparation or the quasi-drug containing the agent for promoting ceramide synthesis of the present invention are not limited. Specific dosage forms include, for example, capsules, tablets, powders, granules, and liquids.

The dosage of the agent for promoting ceramide synthesis of the present invention may be determined in consideration of the patient age, sex, weight, mode of administration, dose, and the like. Modes of administration include application, oral administration, and intravascular injection (infusion).

For example, in the case of application, the ionized alkaline water containing 0.001 to 1.5% by mass of a silicon compound and a phosphorus compound may be applied to the skin. In the case of oral administration of the agent for promoting ceramide synthesis gene expression of the present invention, the dosage is preferably about 100 to 200 mL/day of the ionized alkaline water containing 0.001 to 1.5% by mass of a silicon compound and a phosphorus compound. In the case of intravascular injection of the agent for promoting ceramide synthesis gene expression of the present invention, the dosage is preferably about 100 to 500 mL/day of the ionized alkaline water containing 0.001 to 1.5% by mass of a silicon compound and a phosphorus compound.

The composition such as a pharmaceutical preparation that contains the agent for promoting ceramide synthesis of the present invention promotes the expression of ceramide synthesis genes and thereby effectively promotes ceramide synthesis, and thus, is effective for preventing or treating dermatitis, or ameliorating a skin damaged site, for example. The pharmaceutical preparation or the quasi-drug containing the agent for promoting ceramide synthesis of the present invention may be used as an agent for preventing or treating atopic dermatitis, acne, burns, wounds, gangrene, bedsore, scabies, pyoderma, shingles, scalp eczema, epidermal cyst, herpes simplex, varicella, malignant skin tumors, and athlete's foot, for example. The cosmetic preparation containing the agent for promoting ceramide synthesis of the present invention may be used as a skin moisturizer, a skin dryness inhibitor, a skin roughness inhibitor, a sunburn repair agent, a whitening agent, and the like.

The present invention includes use of ionized alkaline water obtained by electrolyzing an aqueous solution comprising a mineral salt containing silicon and phosphorus, for promoting ceramide synthesis.

Moreover, the present invention includes a method for promoting ceramide synthesis, the method comprising using ionized alkaline water obtained by electrolyzing an aqueous solution comprising a mineral salt containing silicon and phosphorus.

Furthermore, the present invention includes a method for producing an agent for promoting ceramide synthesis, comprising the step of electrolyzing an aqueous solution comprising a mineral salt containing silicon and phosphorus.

### Examples

The present invention will be hereinafter described in more detail with examples; however, the technical scope of the present invention is not limited to these examples.

### (Example 1)

Ionized alkaline water was produced as follows, using the ionized alkaline water producing apparatus 101 as described above.

Deionized water was prepared as raw material water. The deionized water was produced by removing impurities from tap water using an ion exchange resin (from Samson Co., Ltd.). An aqueous solution obtained by adding 1% by mass of a mineral salt containing silicon and phosphorus collected from seawater in Japan to the deionized water was introduced into the electrolytic cell 1 through the water conduit 2, and electricity was supplied to the anode 6 and the cathode 7. Electrolysis was performed under the conditions of a voltage of 200 V and an electrical energy of 2500 W. The deionized water was electrolyzed to produce acidic water in the anode chamber 4 and alkaline water in the cathode chamber 5. The acidic water produced in the anode chamber 4 was discharged through the acidic water discharge pipe 8. The alkaline water produced in the cathode chamber 5 was delivered to the alkaline water delivery pipe 9, and the alkaline water passing through the alkaline water delivery pipe 9 was cooled by the cooling device 10 and then introduced into the mixing tank 11.

In the mixing tank 11, the stirring device 14 mixed the cooled alkaline water with a mineral salt containing silicon and phosphorus collected from seawater in Japan, supplied from the raw material liquid reservoir 12. The mineral salt containing silicon and phosphorus collected from seawater in Japan, in the raw material liquid reservoir 12, was adjusted by the inflow adjusting device 13 such that the content of the mineral salt containing silicon and phosphorus collected from seawater in Japan, in the mixture in the mixing tank 11, was 0.3% by mass, and then supplied. The mixture was stored for 24 hours at a pressure of 294 kPa and a temperature of 4 ± 3°C. During storage, electrons were discharged to the mixture from the cathode terminal 100.

Thereafter, the ionized alkaline water was delivered through the outlet pipe 16, and the pH of the ionized alkaline water was measured by the pH measuring means 17. Then, the ionized alkaline water was drawn through the ionized alkaline water outlet pipe 18.

The ionized alkaline water of Example 1 drawn through the ionized alkaline water outlet pipe was subjected to elemental analysis using ICP optical emission spectrometry (product name: SPECTRO ARCOS II from the manufacturer, Hitachi High-Tech Science Corporation).

The results revealed that the ionized alkaline water of Example 1 contained 100 mass ppm of silicon and 760 mass ppm of phosphorus. More particularly, the ionized alkaline water was found to also contain 7500 mass ppm of sodium, 2700 mass ppm of potassium, 0.2 mass ppm of calcium, and 0.1 mass ppm or less of magnesium.

Since ICP optical emission spectrometry described above cannot detect gases such as chlorine, the ionized alkaline water of Example 1 (sample 1) drawn through the electrolyzed water outlet pipe was separately subjected to measurement using combustion-coulometric titration as described below, at Japan Food Research Laboratories.

Combustion-coulometric titration was performed using a model TSX-10 chlorine/sulfur analyzer from Nittoseiko Analytech Co., Ltd., by combusting about 10 mg of the sample 1, and calculating the chloride ion concentration by coulometric titration. This measurement gives the amount of chlorine in the sample, including both organic chlorine and inorganic chlorine. The amount of organic chlorine was obtained by subtracting the amount of inorganic chlorine from the total amount of chlorine. The amount of inorganic chlorine was obtained by extracting 5 g of the sample with hot water, and measuring chlorine ions in the extract by ion chromatography.

The results revealed that the ionized alkaline water of Example 1 contained 410 mg/kg of chlorine.

### <Measurement of pH>

The pH of the ionized alkaline water obtained in Example 1 was measured with a pH meter (HORIBA F-74SP from HORIBA). As a result, the pH value was 12.

### <Measurement of Surface Tension>

The surface tension of the ionized alkaline water obtained in Example 1 was measured using the Wilhelmy method.

The results revealed that the ionized alkaline water obtained in Example 1 had a surface tension of about 65.41 mN/m (20°C). Using the same method, the surface tension of purified water was measured, and the surface tension was found to be about 72.96 mN/m (20°C). Thus, the surface tension of the ionized alkaline water comprising a mineral salt containing silicon and phosphorus used in the present invention was lower than the surface tension of the purified water by about 7.55 mN/m.

### (Example 2)

Ionized alkaline water was produced as in Example 1, except that the seawater in Japan used as the raw material of the mineral salt containing silicon and phosphorus was replaced by seawater in Okinawa Prefecture.

The resulting ionized alkaline water was subjected to elemental analysis using ICP optical emission spectrometry, as in Example 1. The results revealed that the ionized alkaline water contained 370 mass ppm of silicon and 450 mass ppm of phosphorus. More particularly, the ionized alkaline water was found to also contain 6900 mass ppm of sodium, 1 mass ppm or less of potassium, 0.9 mass ppm of calcium, and 0.3 mass ppm or less of magnesium.

Furthermore, the amount of chlorine contained in the ionized alkaline water obtained in Example 2 was measured using combustion-coulometric titration, at Japan Food Research Laboratories, as in Example 1. The results revealed that the ionized alkaline water of Example 2 contained 410 mg/kg of chlorine.

### (Test Example 1)

The ionized alkaline water produced in Example 1 was examined for its effect on the expression of enzyme genes involved in ceramide synthesis. It should be noted that, on the actual skin, the ionized alkaline water is neutralized by sebum or sweat on the skin or collected in the stratum corneum before it reaches the keratinocytes. Thus, in Test Example 1, the ionized alkaline water of Example 1 was diluted 20-fold, and evaluation was performed using ionized alkaline water having concentrations calculated with this concentration as a reference (100%). The same applies to Test Examples 2 and 3.

Among enzymes involved in ceramide synthesis, the ultra-long chain fatty acid elongase 4 (ELOVL4), the ultra-long chain fatty acid elongase 7 (ELOVL7), the ceramide synthetase 3 (CerS3), and the ceramide synthetase (SPTLC2) were evaluated for variations in their expression. The primers for the target genes were designed based on the sequences reported by human RT-PCR (Shirakura et al.: Lipid in Health and Disease, 11,108 (2012).).

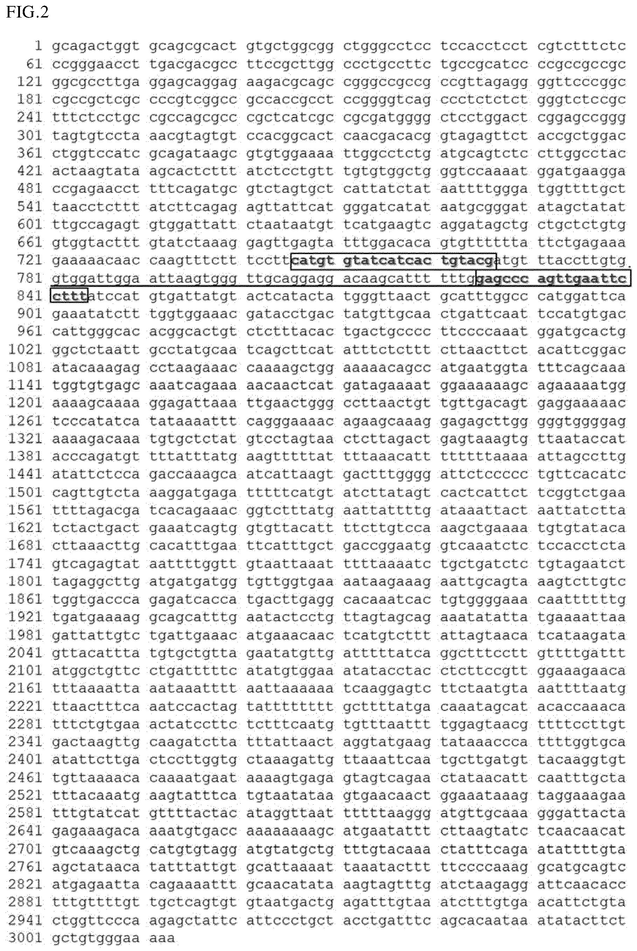

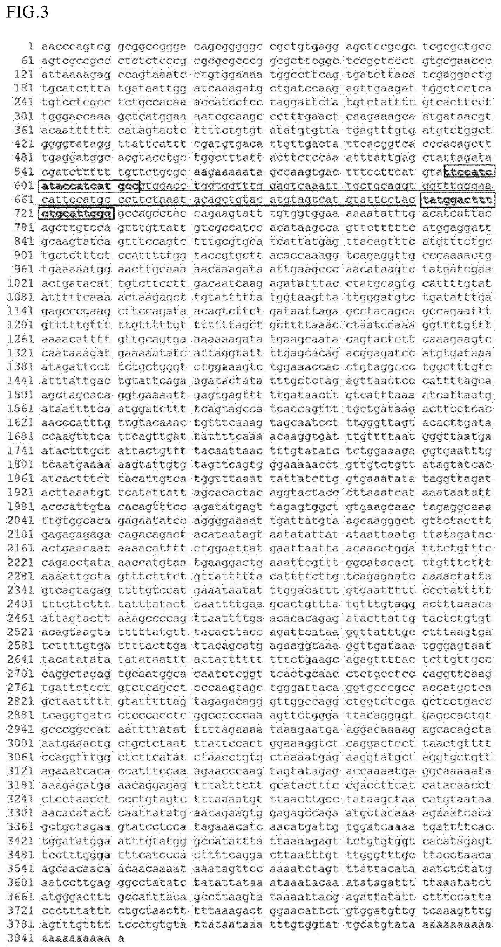

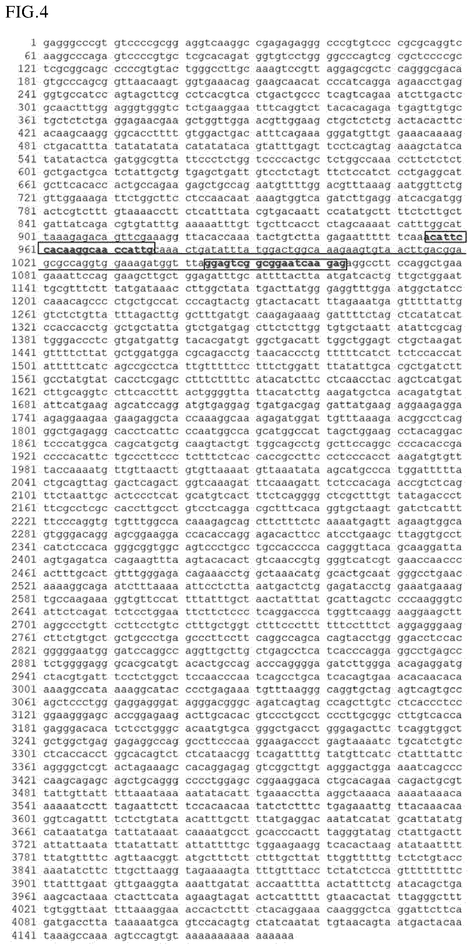

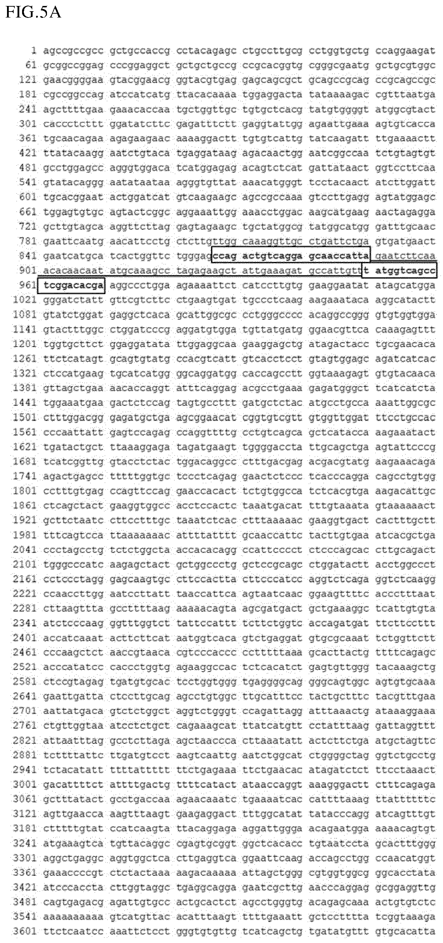

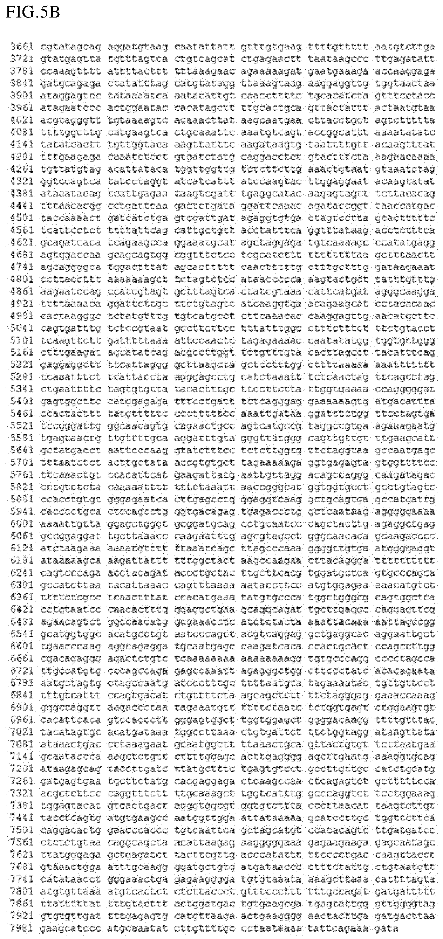

Forward and reverse primers were synthesized for each of human ELOVL4, ELOVL7, CerS3, and SPTLC2. The primers were obtained by outsourcing the synthesis of PCR primers to FASMAC. Fig. 2 shows the gene sequence of human ELOVL4 (SEQ ID NO: 1). Fig. 3 shows the gene sequence of human ELOVL7 (SEQ ID NO: 2). Fig. 4 shows the gene sequence of human CerS3 (SEQ ID NO: 3). Figs. 5A and 5B show the gene sequence of human SPTLC2 (SEQ ID NO: 4). Table 1 shows the sequence of the forward primer of ELOVL4 (SEQ ID NO: 5) and the sequence of the reverse primer of ELOVL4 (SEQ ID NO: 6); the sequence of the forward primer of ELOVL7 (SEQ ID NO: 7) and the sequence of the reverse primer of ELOVL7 (SEQ ID NO: 8); the sequence of the forward primer of CerS3 (SEQ ID NO: 9) and the sequence of the reverse primer of CerS3 (SEQ ID NO: 10); the sequence of the forward primer of SPTLC2 (SEQ ID NO: 11) and the sequence of the reverse primer of SPTLC2 (SEQ ID NO: 12); and the sequence of the forward primer of β-actin (endogenous control) (SEQ ID NO: 13) and the sequence of the reverse primer of β-actin (SEQ ID NO: 14). In a PCR reaction, the primers bind to the complementary strands of forward and reverse primers, and DNA fragments are amplified by the PCR reaction between the two primers. In Figs. 2 to Figs. 5A and 5B, the sections in bold surrounded by a black frame in each sequence indicate the sequences to which the primers bind (complementary strands of the forward primer and the reverse primer). The underlined portion indicates the sequence amplified by the PCR reaction.

**[Table 1]**

| Table 1 | Nucleotide Sequences of Primers | |
|---|---|---|
| Amplified Genes | Forward Primer | Reverse Primer |
| ELOVL4 | 5'-CATGTGTATCATCACTGTACG-3' | 5'-AAAGGAATTCAACTGGGCTC-3' |
| ELOVL7 | 5'-TTCCATCATACCATCATGCC-3' | 5'-CCCAATGCAGAAAGTCCATA-3' |
| CerS3 | 5'-ACATTCCACAAGGCAACCATTG-3' | 5'-CTCTTGATTCCGCCGACTCC-3' |
| SPTLC2 | 5'-CCAGACTGTCAGGAGCAACCATTA-3' | 5'-TCGTGTCCGAGGCTGACCATA-3' |
| β-Actin | 5'-AGTCCTGTGGCATCCACGAAAC-3' | 5'-GCAGTGATCTCCTTCTGCATCC-3' |

### <Reagents>

- Formazan (MTT); from Dojindo Laboratories Co., Ltd.
- Microtest plate for cell culture: 96-well, flat bottom, low evaporation-type; from FALCON
- Fetal bovine serum: FBS; from Biosera
- Dulbecco's modified eagle medium: DMEM; from WAKO
- TriPure isolation reagent (TriPure Reagent); from Takara Bio Inc.
- Random primer; from Takara Bio Inc.
- Reverse transcriptase: Revatra Ace; from TOYOBO
- THUNDERBIRD (registered trademark) SYBR qPCR Mix; from TOYOBO

### <Apparatuses>

- Microplate reader: ARVO MX1420 MULTIABEL COUNTER from WALLAC was used.
- Real time PCR: StepOne Real Time PCR (48 well) from Thermo Fisher Scientific was used.

### <Preparation of DNA>

Normal human epidermal keratinocytes (NHEKs) were plated in a 6-well plate at a density of 1 × 10⁵ cells per well and cultured for 24 hours. Then, DMEM containing 10%, 20%, or 100% (v/v%) of the ionized alkaline water and 10% FBS was added to each well, and the cells were cultured for 72 hours. As a control, DMEM (containing 10% FBS) not containing the ionized alkaline water was used. After 72 hours of culture, total RNA was extracted using the TriPure Reagent from Takara Bio Inc., as instructed by the attached manual. The mRNAs of ELOVL4, ELOVL7, CerS3, and SPTLC2 were quantified by real time-PCR, using cDNAs that have been prepared. β-actin was used as an endogenous control. Data analysis was performed using the comparative ΔΔCt method.

### <Measurement Conditions>

Real time-PCR was performed using the StepOne (Thermo Fisher Scientific) and also using THUNDERBIRD (registered trademark) SYBR qPCR Mix from TOYOBO.

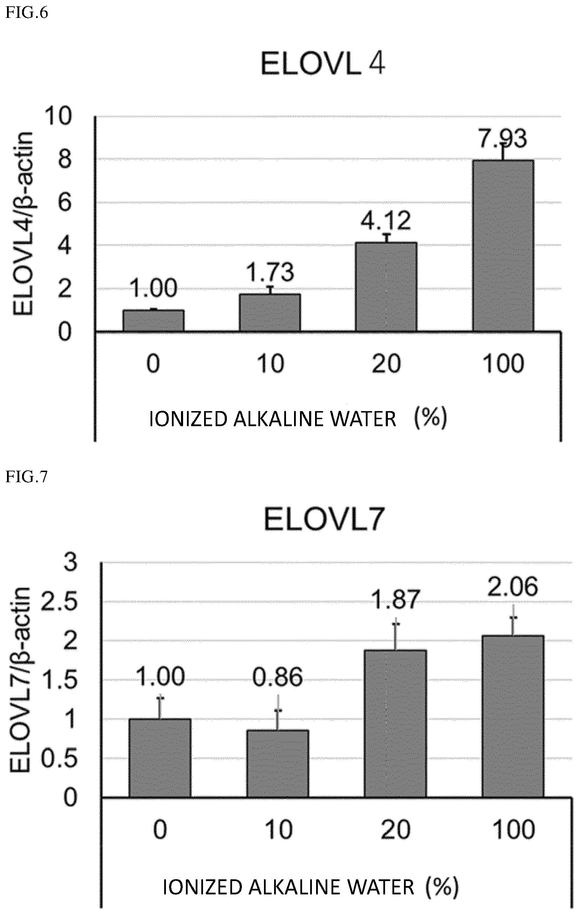

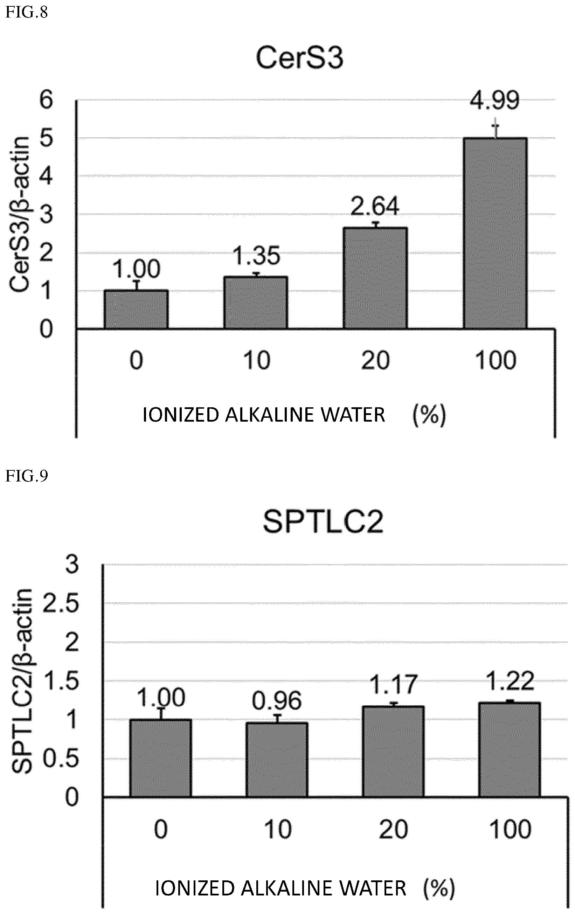

Fig. 6 shows the results of the mRNA expression of ELOVL4 with the ionized alkaline water; Fig. 7 shows the results of the mRNA expression of ELOVL7 with the ionized alkaline water; Fig. 8 shows the results of the mRNA expression of CerS3 with the ionized alkaline water; and Fig. 9 shows the results of the mRNA expression of SPTLC2 with the ionized alkaline water. The mRNA expression level is expressed in terms of relative value with the expression level of β-actin taken as 1.

Fig. 6 shows that the ionized alkaline water promoted the mRNA expression of ELOVL4. As shown in Fig. 7, the mRNA expression of ELOVL7 was also observed. Fig. 8 shows that the ionized alkaline water promoted the mRNA expression of CerS3. Fig. 9 shows that the ionized alkaline water promoted the mRNA expression of SPTLC2.

### (Test Example 2)

Variations in the expression of the ELOVL4 and CerS3 proteins were evaluated by Western blotting with specific antibodies.

More particularly, normal human epidermal keratinocytes (NHEKs) were plated in a 6-well plate at a density of 1 × 10⁵ cells per well and cultured for 24 hours. Then, DMEM containing 10%, 20%, or 100% (v/v%) of the ionized alkaline water and 10% FBS was added to each well, and the cells were cultured for 72 hours. As a control, DMEM (containing 10% FBS) not containing the ionized alkaline water was used. After 72 hours of culture, the culture was washed with phosphate buffer. Thereafter, the cells were collected as a cell lysate (50 mM Tris-HCl, 150 mM NaCl, 1% NP-40, 1 mM EDTA, pH 7.4) and centrifuged (10,000 rpm, 10 min, 4°C), and the supernatant was obtained as a protein extract. The protein extract was subjected to SDS-PAGE (SDS-polyacrylamide gel electrophoresis) to separate the protein, and then the protein was transferred to a nitrocellulose membrane, and the membrane was blocked in phosphate buffer containing 2% skim milk. For detection of CerS3, rabbit anti-CerS3 antibody (Cusabio, 2000-fold diluted) was used; for detection of ELOVL4, rabbit anti-ELOVL4 antibody (Cusabio, 1,000-fold diluted) was used; and for detection of β-actin, mouse anti-β-actin antibody (Wako Pure Chemical, 3000-fold diluted) was used; and each antibody was reacted with the nitrocellulose membrane. The nitrocellulose membrane was thoroughly washed with phosphate buffer, and then the target protein was labeled with peroxidase using peroxidase-labeled goat anti-rabbit IgG antibody (KPL; 5,000-fold diluted) or peroxidase-labeled goat anti-mouse IgG antibody (Jackson; 3,000-fold diluted). The detection was performed using a luminescent reagent (ImmunoStar LD; Wako Pure Chemical), and the membrane was imaged using the LuminoGraph (Atto).

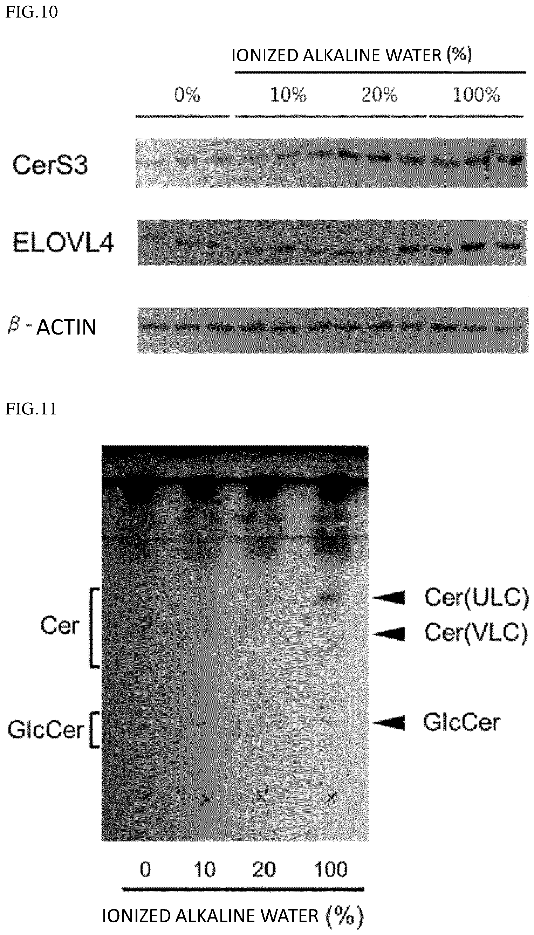

The results are shown in Fig. 10.

Fig. 10 shows that the ionized alkaline water promoted the expression of the CerS3 protein in a concentration-dependent manner.

### (Test Example 3)

Evaluation as to whether the ionized alkaline water can actually promote the synthesis of ceramide (Cer) and glucosylceramide (GlcCer) was performed by thin-layer chromatography (TLC).

More particularly, normal human epidermal keratinocytes (NHEKs) were plated in a 6-well plate at a density of 1 × 10⁵ cells per well and cultured for 24 hours. Then, DMEM containing 10%, 20%, or 100% (v/v%) of the ionized alkaline water and 10% FBS was added to each well, and the cells were cultured for 72 hours. As a control, DMEM (containing 10% FBS) not containing the ionized alkaline water was used. After 48 hours of culture, the cells were washed with phosphate buffer, and then lipids in the cells were shaken in a chloroform : methanol (1:2, v/v) extraction solution for 1 hour, and the supernatant was collected. Thereafter, a chloroform : methanol : water (1:2:0.5, v/v) extraction solution was added to the remaining residue, and the supernatant was collected. A 2.5% (w/v) aqueous potassium chloride solution was added to the extracted sample, the mixture was centrifuged (5 min, 900×g, room temperature), and then the lower layer was collected in a fresh tube. Lipids remaining in the upper layer were collected by adding chloroform and stirring and then harvesting the lower layer formed after centrifugation (5 min, 900×g, room temperature). The lipid fraction was dried to solid in a centrifugal evaporator and dissolved in chloroform : methanol (2:1, v/v), and then stored at -20°C until analysis.

Thin layer chromatography was performed in the following procedure: (1) A TLC plate was developed with chloroform : methanol : water (40:10:1, v/v) to 2 cm from the point where the sample was added, and then dried. (2) The TLC plate was developed with chloroform : methanol : water (40:10:1, v/v) to 5 cm from the point where the sample was added, and then dried. (3) The TLC plate was developed with chloroform : methanol : acetic acid (47:2:0.5, v/v) to 1.5 cm from the top edge of the TLC plate, and then dried. (4) The TLC plate was developed with hexane : diethyl ether : acetic acid (65:35:1, v/v) to 0.5 cm from the top edge of the TLC plate, and then dried. For detection of lipids on the developed TLC plate, the TLC plate was thoroughly sprayed with an 8% (v/v) aqueous phosphoric acid solution in which 3% (w/v) copper sulfate was dissolved and then heated on a hot plate at 180°C.

The results are shown in Fig. 11. In Fig. 11, "VLC" designates very long chains (usually, carbon chains longer than 20 carbons), and "ULC" designates ultra-long chains (usually, carbon chains with 26 carbons or more).

Next, the Rf values of major spots in the GlcCer region and the Cer region in Fig. 11 are shown in Table 2.

**[Table 2]**

| Table 2 | Ionized alkaline water (%) | 0 | 10 | 20 | 100 |
|---|---|---|---|---|---|
| Rf Value | GlcCer Region | 0.2 | 0.2 | 0.2 | 0.21 |
| | Cer Region | 0.52 | 0.52 | 0.53 | 0.63 |

For measurement of the color intensity of each region, the images were obtained using the image capturing apparatus LumiCube (from Liponics, Inc.) and then subjected to the measurement using the image processing software ImageJ (NIH).

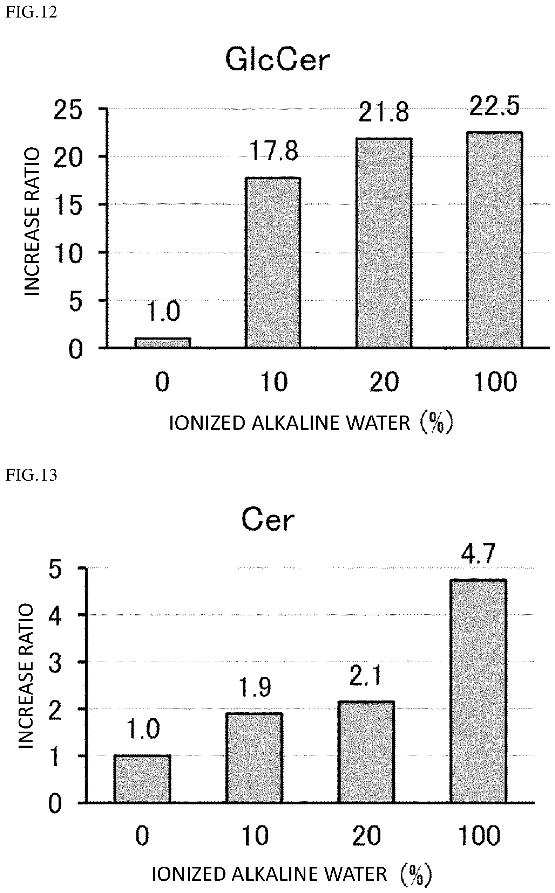

Table 3 and Fig. 12 show the increase ratio of the color intensity of the GlcCer region when the color intensity of the GlcCer region at 0% of the ionized alkaline water (when cultured in the ionized alkaline water-free medium (DMEM)) is taken as 1. Table 3 and Fig. 13 show the increase ratio of the color intensity of the Cer region when the color intensity of the Cer region at 0% of the ionized alkaline water is taken as 1.

**[Table 3]**

| Table 3 | GlcCer | | Cer | |
|---|---|---|---|---|
| Ionized alkaline water (%) | Color Intensity | Increase Ratio | Color Intensity | Increase Ratio |
| 0 | 10.6 | 1.0 | 206.6 | 1.0 |
| 10 | 188.8 | 17.8 | 392.4 | 1.9 |
| 20 | 232.1 | 21.8 | 444.2 | 2.1 |
| 100 | 238.9 | 22.5 | 979.3 | 4.7 |

FIG. 11 has confirmed that as the concentration of the ionized alkaline water increases, the spot size is larger, and the color is darker, in the GlcCer region and the Cer region (particularly the Cer (ULC) region). Furthermore, Table 3 shows that the higher the concentration of the ionized alkaline water, the higher the increase ratios of the color intensities of the GlcCer region and the Cer region. These results indicate that the higher the concentration of the ionized alkaline water, the greater the amount of ceramides synthesized, revealing that the ionized alkaline water promoted the synthesis of GlcCer and Cer in a concentration-dependent manner and increased the intracellular contents of GlcCer and Cer.

Fig. 11 and Table 2 have also confirmed that the higher the concentration of the ionized alkaline water, the higher the Rf values (i.e., the longer the distance of development) of the spots in the GlcCer region and the Cer region (particularly the Cer (ULC) region). Table 3 shows that the higher the concentration of the ionized alkaline water, the higher the increase ratios of the color intensities of the GlcCer region and the Cer region. These results indicate that the higher the concentration of the ionized alkaline water, the more the synthesis of fatty acids with a long carbon chain length is promoted, and that, because lipophilicity increases (i.e., hydrophobicity increases) as the fatty acid length increases, the distance of development increases, and the spot is detected at a higher point.

The foregoing suggests that the ionized alkaline water promotes the synthesis of a ceramide (acylceramide) composed of ultra-long chain fatty acids in keratinocytes.

### Reference Signs List

1: electrolytic cell
2: water conduit
3: diaphragm
4: anode chamber
5: cathode chamber
6: anode
7: cathode
8: acidic water discharge pipe
9: alkaline water delivery pipe
10: cooling device
11: mixing tank
12: raw material liquid reservoir
13: inflow adjusting device
14: stirring device
15: thermal insulating means
16: outlet pipe
17: pH measuring means
18: ionized alkaline water outlet pipe
19: alcohol reservoir
20: alcohol mixing tank
21: ionized alkaline water mixture outlet pipe
22: isolated chamber
100: cathode electron
101: ionized alkaline water producing apparatus

## Claims

1. An agent for promoting ceramide synthesis comprising ionized alkaline water obtained by electrolyzing an aqueous solution comprising a mineral salt containing silicon and phosphorus.

2. The agent for promoting ceramide synthesis according to claim 1, wherein the ionized alkaline water contains 0.4 to 400 mass ppm of the silicon and 400 to 800 mass ppm of the phosphorus.

3. The agent for promoting ceramide synthesis according to claim 1 or 2, wherein the ionized alkaline water further contains at least one mineral selected from the group consisting of calcium, potassium, sodium, and magnesium.

4. The agent for promoting ceramide synthesis according to any one of claims 1 to 3, wherein the ionized alkaline water further contains chlorine.

5. The agent for promoting ceramide synthesis according to any one of claims 1 to 4, wherein the ionized alkaline water has a pH of 10 to 12.5.

6. The agent for promoting ceramide synthesis according to any one of claims 1 to 5, wherein the ionized alkaline water has a surface tension of 55 to 68 mN/m.

7. The agent for promoting ceramide synthesis according to any one of claims 1 to 6, wherein the ionized alkaline water is obtained by:
an electrolyzing step of electrolyzing an aqueous solution comprising water and a mineral salt containing silicon and phosphorus; and
an electron supplying step of mixing cathode side-alkaline water obtained in the preceding step with a mineral salt containing silicon and phosphorus to obtain a mixture, and supplying electrons to the mixture.

8. The agent for promoting ceramide synthesis according to any one of claims 1 to 7, wherein the agent for promoting ceramide synthesis comprises an agent for promoting ceramide synthesis gene expression.

9. The agent for promoting ceramide synthesis according to any one of claims 1 to 8, wherein the agent for promoting ceramide synthesis gene expression comprises at least one agent for promoting gene expression selected from the group consisting of an agent for promoting ELOVL4 gene expression, an agent for promoting ELOVL7 gene expression, an agent for promoting CerS3 gene expression, and an agent for promoting SPTLC2 gene expression.

10. A pharmaceutical preparation comprising the agent for promoting ceramide synthesis according to any one of claims 1 to 9.

11. A skin external preparation comprising the agent for promoting ceramide synthesis according to any one of claims 1 to 9.

12. A cosmetic preparation comprising the agent for promoting ceramide synthesis according to any one of claims 1 to 9.

13. A food or beverage composition comprising the agent for promoting ceramide synthesis according to any one of claims 1 to 9.

14. Use of ionized alkaline water obtained by electrolyzing an aqueous solution comprising a mineral salt containing silicon and phosphorus, for promoting ceramide synthesis.

15. A method for promoting ceramide synthesis,
the method comprising using ionized alkaline water obtained by electrolyzing an aqueous solution comprising a mineral salt containing silicon and phosphorus.
